# EUROPEAN PATENT APPLICATION

(11) **EP 2 671 510 A1**
(43) Date of publication of application: **11.12.2013**
(21) Application number: 12742458.8
(22) Date of filing: 26.01.2012
(51) Int. Cl.: A61B 5/16, A61B 5/01

(54) **ESTIMATION DEVICE, ESTIMATION SYSTEM, ESTIMATION METHOD AND PROGRAM FOR JET LAG SYMPTOMS**

(30) Priority: 03.02.2011 JP 2011022064
(71) Applicant: Sony Corporation, Tokyo 108-0075 (JP)
(72) Inventor: SANO, Akane, Tokyo 108-0075 (JP); SAZUKA, Naoya, Tokyo 108-0075 (JP)
(74) Representative: Körber, Martin Hans
(86) International application number: PCT/JP2012/051724
(87) International publication number: WO 2012/105422

(57) **Abstract**

Provided is an estimation device for a jet lag symptom, including an acquiring unit acquiring an estimation criterion set in a manner that each of feature quantities of a physiological index of a core body temperature system and a physiological index of an activity system is classified according to the severity of the jet lag symptom, and a feature quantity range is specified according to a classification of the severity of the jet lag symptom, an extracting unit extracting the feature quantities of the physiological index of the core body temperature system and the physiological index of the activity system on a subject, and an estimating unit determining which feature quantity range of the estimation criterion includes a feature quantity of a physiological index of the subject, and estimates a jet lag symptom of the subject.

## Description

### Technical Field

The present disclosure relates to an estimation device, an estimation system, an estimation method, and a program for a jet lag symptom.

### Background Art

Jet lag (in the broad sense) deviates biological rhythms and affects a physical condition due to movement involving a time difference, shift working, an irregular lifestyle (lifestyle habits in which working days greatly differ in a sleep phase from non-working days or irregular sleep-wake habits), or the like. Jet lag causes symptoms such as sleep disorders, persistent fatigue, and the like. When the physical condition is affected by the symptoms of jet lag, the physical ability decreases, and an unexpected accident may occur. For this reason, from a point of view of physical condition management, it is desirable to objectively determine the presence or absence of jet lag or quantitatively evaluate a severity of jet lag symptoms.

However, the jet lag symptoms are subject to individual differences, an age gap, a difference in a moving direction of east and west, and the like. For example, it is known that there is an individual difference of 1.7 days to 17.9 days in the duration of jet lag symptoms (Reference 1). For this reason, it is difficult to make an objective determination or quantitative evaluation, and the determination or evaluation is merely performed subjectively or qualitatively.

### Summary of Invention

### Technical Problem

Meanwhile, among biological rhythms having various cycles, body temperature, particularly, biological rhythms such as body temperature, blood pressure, a heart rate, and sleep and wakefulness are known as biological rhythms having a circadian cycle. Further, an adapting time (a time necessary until deviation of biological rhythms caused by a time difference adapts to actual living hours) of biological rhythms is known to significantly differ between a physiological index of a core body temperature system and a physiological index of an activity (sleep and wakefulness) system. For example, deviation of biological rhythm caused by a time difference of five hours or more is known to be about two weeks in the core body temperature and two or three days in the heart rate and the sleep and wakefulness (References 2 and 3). Further, the jet lag symptoms are known to be severest on a third or fourth day after jet lag is caused (Reference 4).

Further, a difference in an adapting time between biological rhythms causes a phenomenon of internal desynchronization in which a plurality of biological rhythms have different cycles in the same living body, causing so-called jet lag (in the broad sense) that causes the living body to be in various bad conditions (Reference 5). For this reason, it is difficult to quantitatively determine the presence or absence of jet lag or quantitatively evaluate a severity of jet lag symptoms using a single physiological index.

It is desirable to provide an estimation device, an estimation system, an estimation method, and a program for jet lag symptoms, which are capable of objectively determining the presence or absence of jet lag and quantitatively evaluating a severity of jet lag symptoms.

### Solution to Problem

According to an embodiment of the present disclosure, there is provided an estimation device for a jet lag symptom, including an acquiring unit that acquires an estimation criterion set in a manner that, for a plurality of samples that differ in a severity of a jet lag symptom, each of a feature quantity of a physiological index of a core body temperature system and a feature quantity of a physiological index of an activity system is classified according to the severity of the jet lag symptom, and a feature quantity range is specified according to a classification of the severity of the jet lag symptom, an extracting unit that extracts the feature quantity of the physiological index of the core body temperature system and the feature quantity of the physiological index of the activity system on a subject, and an estimating unit that determines which feature quantity range of the estimation criterion includes a feature quantity of a physiological index of the subject on each of the physiological index of the core body temperature system and the physiological index of the activity system, and estimates a jet lag symptom of the subject.

The estimating unit may estimate the severity of the jet lag symptom of the subject to be severe when the feature quantity of the physiological index of the core body temperature system is included in a feature quantity range of a sample having a jet lag symptom, and the feature quantity of the physiological index of the activity system is included in a feature quantity range of a feature quantity of a sample having no jet lag symptoms.

The acquiring unit may acquire an estimation criterion set in a manner that feature quantities of the plurality of samples are classified into a feature quantity range of a sample having a jet lag symptom and a feature quantity range of the sample having no jet lag symptoms, the feature quantities of the plurality of samples are classified into three or more according to the severity of the jet lag symptom based on a center of the feature quantity range of the sample having the jet lag symptom, and a sub feature quantity range is specified according to the severity of the jet lag symptom. The estimating unit may determine which sub feature quantity range of the estimation criterion includes the feature quantity of the physiological index of the subject, and may estimate the jet lag symptom of the subject.

The acquiring unit may acquire an estimation criterion set in a manner that the feature quantities of the plurality of samples are classified into three or more based on subjective evaluation on the severity of the jet lag symptom based on the sample, and a sub feature quantity range is specified according to the severity of the jet lag symptom. The estimating unit may determine which sub feature quantity range of the estimation criterion includes the feature quantity of the physiological index of the subject, and may estimate the jet lag symptom of the subject.

The acquiring unit may acquire an estimation criterion set in a manner that feature quantities of a plurality of samples including the subject are classified according to the severity of the jet lag symptom, and a feature quantity range according to a classification of the severity of the jet lag symptom is specified.

The estimation device for the jet lag symptom may further include an estimation history storing unit that stores an estimation result of the jet lag symptom in association with a time difference condition representing a condition of a time difference serving as a cause of the jet lag symptom, and an estimation history extracting unit that extracts an estimation result suitable for a designated time difference condition from the stored estimation result of the jet lag symptom.

The estimation device may be connected to a managing device that acquires the feature quantity of the physiological index of the subject and a classification result of a feature quantity according to the jet lag symptom from a plurality of estimation devices, specifies a feature quantity range according to a classification of the severity of the jet lag symptom, and sets an estimation criterion. The acquiring unit may acquire the estimation criterion set by the managing device.

The estimation device may be connected to a managing device that acquires a classification result of the feature quantity of the physiological index of the subject, a time difference condition representing a condition of a time difference serving as a cause of the jet lag symptom, and an estimation result of the jet lag symptom from a plurality of estimation devices, and manages the estimation result of the jet lag symptom in association with the classification result of the feature quantity and the time difference condition. The estimation device may further include an estimation history acquiring unit that acquires an estimation result suitable for a designated time difference condition and a classification result of the feature quantity among the managed estimation results of the jet lag symptom.

The extracting unit may extract the feature quantity of the physiological index of the core body temperature system and the feature quantity of the physiological index of the activity system on the plurality of samples that differ in the severity of the jet lag symptom. The estimation device may further include a setting unit that classifies the extracted feature quantity of the physiological index according to the severity of the jet lag symptom on each of the physiological index of the core body temperature system and the physiological index of the activity system, specifies a feature quantity range according to a classification of the severity of the jet lag symptom, and sets an estimation criterion. The acquiring unit may acquire the estimation criterion set by the setting unit.

The estimation criterion may be set for each living style of the sample. The estimating unit may estimate the jet lag symptom of the subject using an estimation criterion according to a living style of the subject.

The estimation device for the jet lag symptom may further include a notifying unit that notifies the subject of the estimation result of the jet lag symptom.

The physiological index of the core body temperature system may be an eardrum temperature, and the physiological index of the activity system is activity.

The estimation device for the jet lag symptom may further include a storage unit that stores the estimation criterion. The acquiring unit may acquire the estimation criterion from the storage unit.

According to another embodiment of the present disclosure, there is provided an estimation system for a jet lag symptom, including a setting device including an extracting unit that extracts a feature quantity of a physiological index of a core body temperature system and a feature quantity of a physiological index of an activity system on a plurality of samples that differ in a severity of a jet lag symptom, and a setting unit that classifies the extracted feature quantity of the physiological index according to the severity of the jet lag symptom on each of the physiological index of the core body temperature system and the physiological index of the activity system, specifies a feature quantity range according to a classification of the severity of the jet lag symptom, and sets an estimation criterion; and an estimation device including an extracting unit that extracts the feature quantity of the physiological index of the core body temperature system and the feature quantity of the physiological index of the activity system on a subject, and an estimating unit that determines which feature quantity range of the estimation criterion includes a feature quantity of a physiological index of the subject on each of the physiological index of the core body temperature system and the physiological index of the activity system, and estimates a jet lag symptom of the subject.

According to another embodiment of the present disclosure, there is provided an estimation method for a jet lag symptom, including acquiring an estimation criterion set in a manner that, for a plurality of samples that differ in a severity of a jet lag symptom, each of a feature quantity of a physiological index of a core body temperature system and a feature quantity of a physiological index of an activity system is classified according to the severity of the jet lag symptom, and a feature quantity range is specified according to a classification of the severity of the jet lag symptom, extracting the feature quantity of the physiological index of the core body temperature system and the feature quantity of the physiological index of the activity system on a subject, and determining which feature quantity range of the estimation criterion includes a feature quantity of a physiological index of the subject on each of the physiological index of the core body temperature system and the physiological index of the activity system, and estimating a jet lag symptom of the subject.

According to another embodiment of the present disclosure, there is provided a program for causing a computer to execute an estimation method for a jet lag symptom, the method including acquiring an estimation criterion set in a manner that, for a plurality of samples that differ in a severity of a jet lag symptom, each of a feature quantity of a physiological index of a core body temperature system and a feature quantity of a physiological index of an activity system is classified according to the severity of the jet lag symptom, and a feature quantity range is specified according to a classification of the severity of the jet lag symptom, extracting the feature quantity of the physiological index of the core body temperature system and the feature quantity of the physiological index of the activity system on a subject, and determining which feature quantity range of the estimation criterion includes a feature quantity of a physiological index of the subject on each of the physiological index of the core body temperature system and the physiological index of the activity system, and estimating a jet lag symptom of the subject.

According to another aspect of the present disclosure, there is provided a program causing a computer to execute the estimation method for the jet lag symptom. Here, the program may be provided using a computer readable recording medium or may be provided through a communication means or the like.

### Advantageous Effects of Invention

According to the present disclosure, it is possible to provide an estimation device, an estimation system, an estimation method, and a program for a jet lag symptom, which are capable of objectively determining the presence or absence of jet lag and quantitatively evaluating a severity of a jet lag symptom.

### Brief Description of Drawings

[Fig. 1]

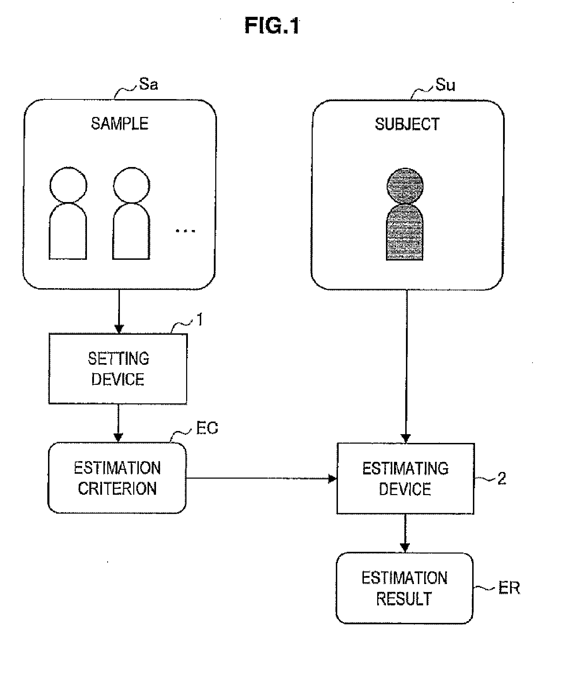

   Fig. 1 is a diagram illustrating an overall configuration of an estimation system for a jet lag symptom according to an embodiment of the present disclosure.
[Fig. 2]

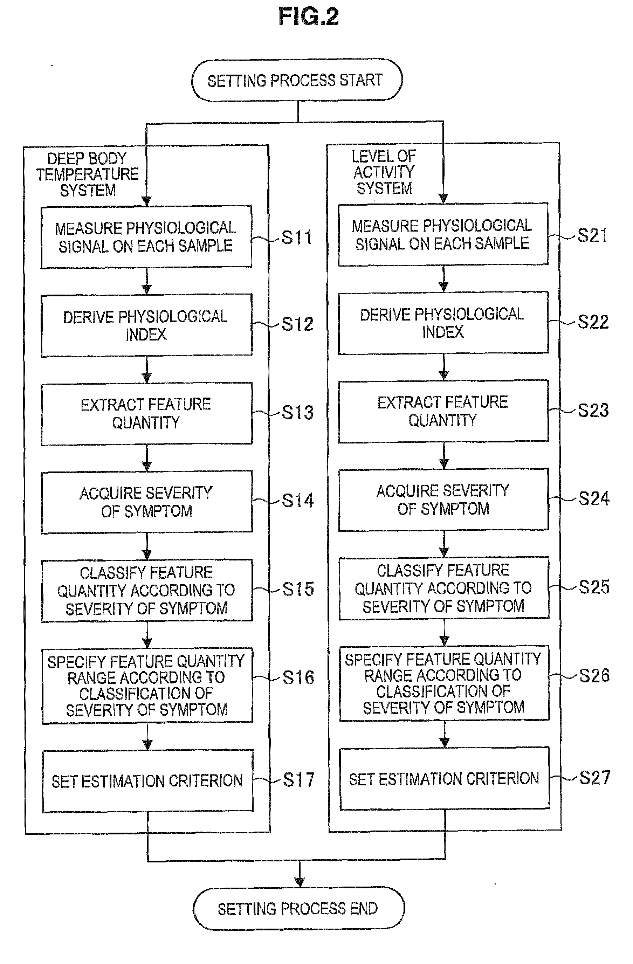

   Fig. 2 is a flowchart illustrating an overall process of an estimation system (1/2).
[Fig. 3]

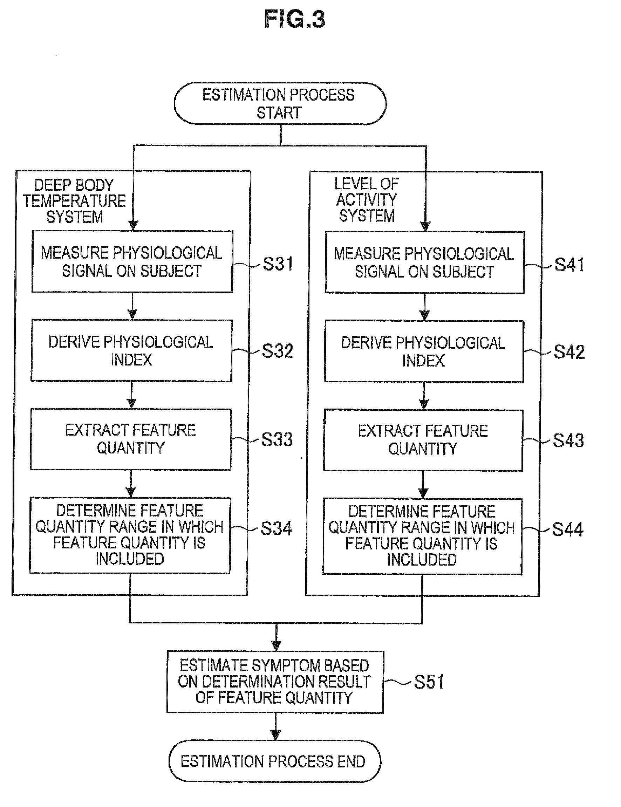

   Fig. 3 is a flowchart illustrating an overall process of an estimation system (2/2).
[Fig. 4]

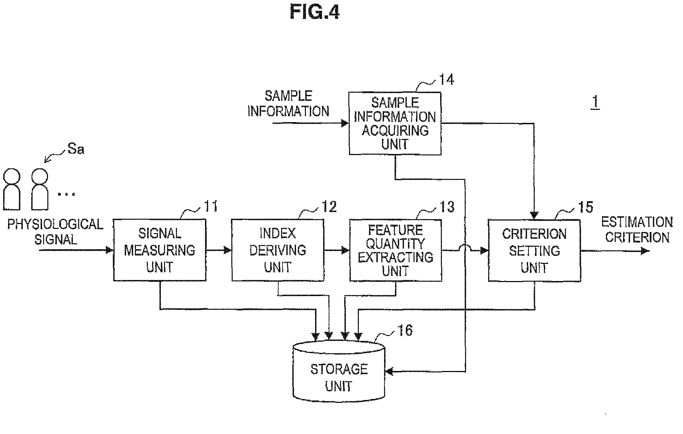

   Fig. 4 is a block diagram illustrating a functional configuration of a setting device.
[Fig. 5]

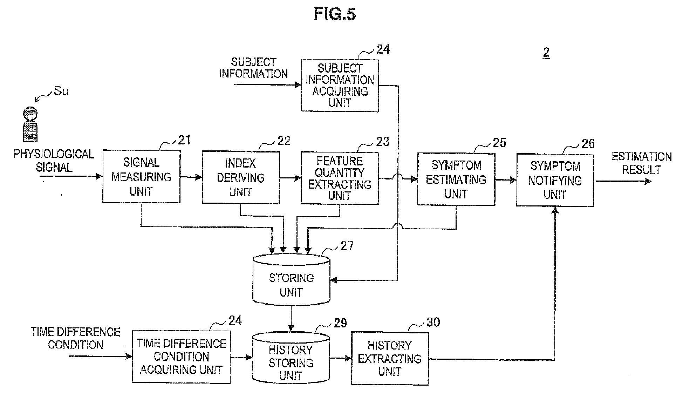

   Fig. 5 is a block diagram illustrating a functional configuration of an estimation device.
[Fig. 6]

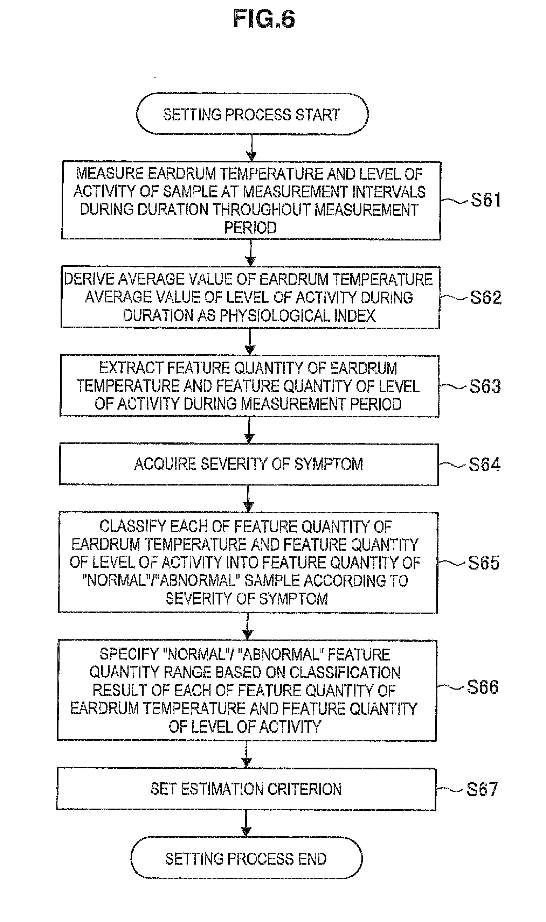

   Fig. 6 is a flowchart illustrating an operation of a setting device.
[Fig. 7]

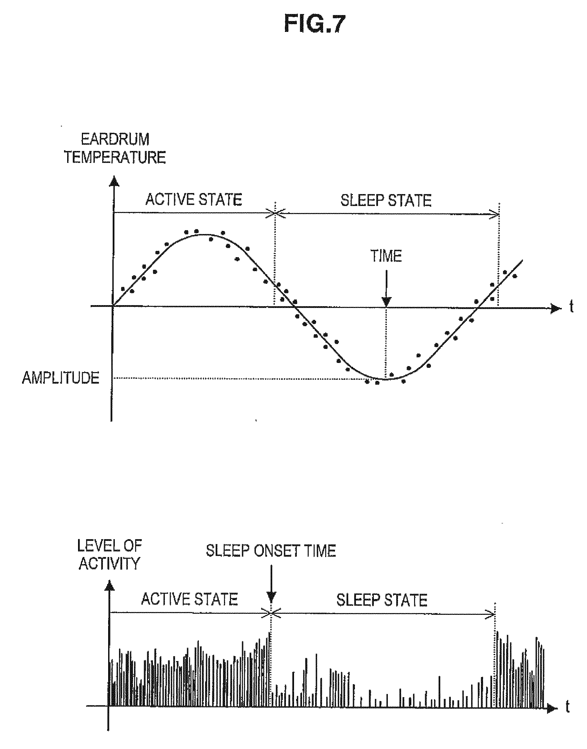

   Fig. 7 is a diagram illustrating an example of a feature quantity of an eardrum temperature and a feature quantity of activity.
[Fig. 8]

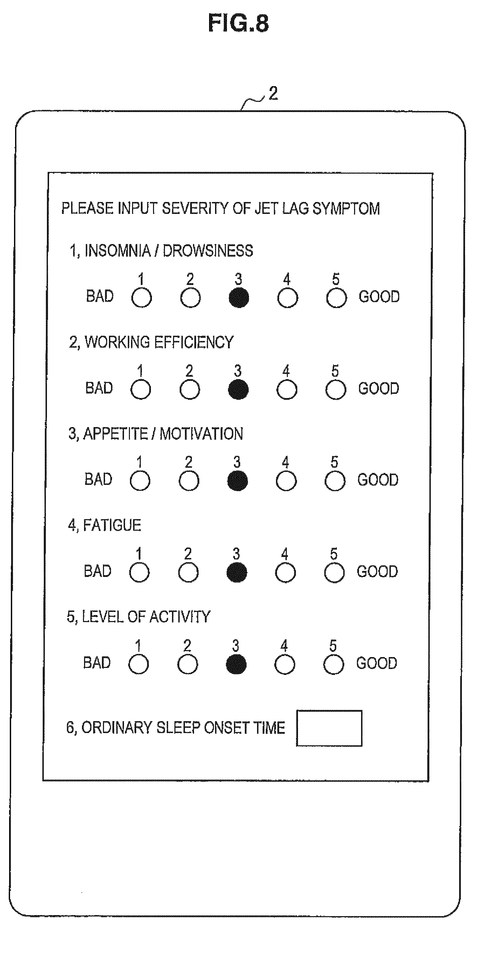

   Fig. 8 is a diagram illustrating an example of a questionnaire for a sample source.
[Fig. 9]

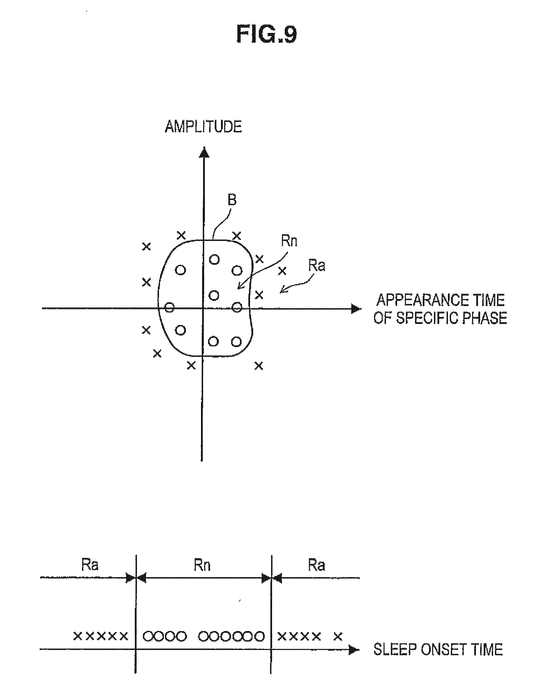

   Fig. 9 is a diagram illustrating an example of specifying the feature quantity range on a feature quantity of an eardrum temperature and a feature quantity of activity.
[Fig. 10]

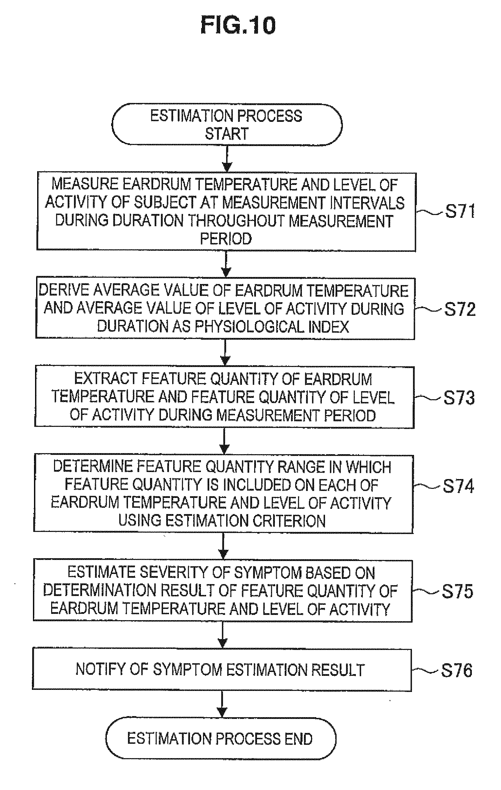

   Fig. 10 is a flowchart illustrating an operation of an estimation device.
[Fig. 11]

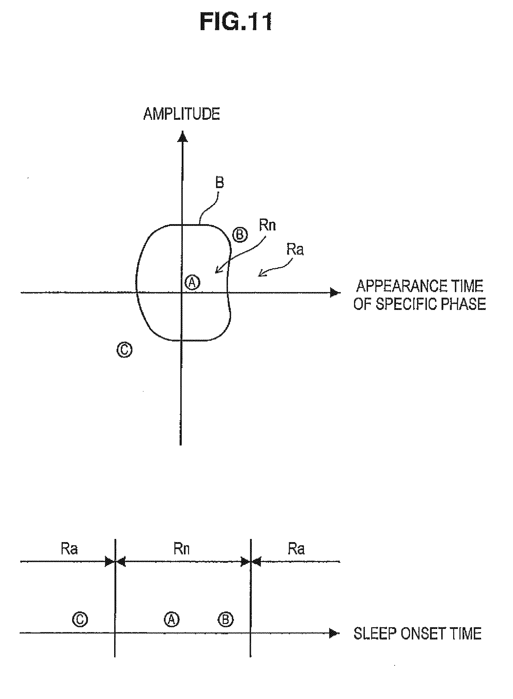

   Fig. 11 is a diagram illustrating an example of determining a feature quantity on a feature quantity of an eardrum temperature and a feature quantity of activity.
[Fig. 12]

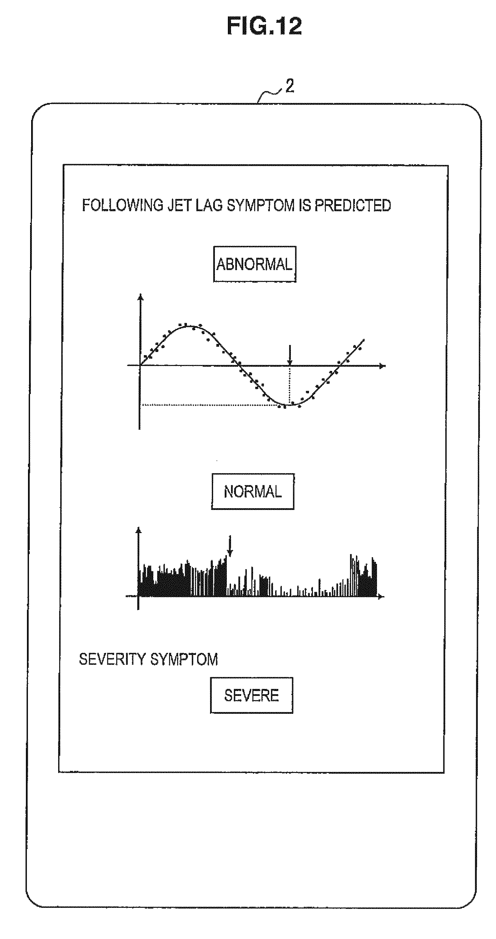

   Fig. 12 is a diagram illustrating an example of notifying of a symptom estimation result.
[Fig. 13]

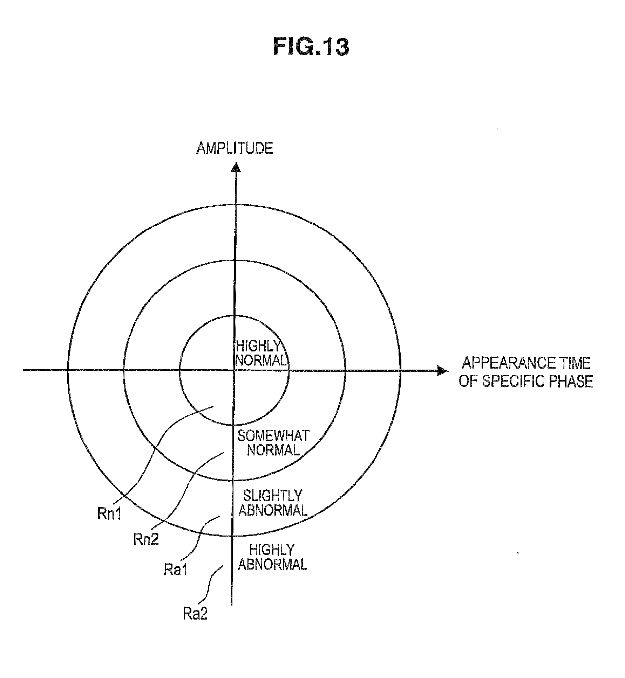

   Fig. 13 is a diagram illustrating an example of an estimation criterion of a feature quantity of an eardrum temperature based on sample information.
[Fig. 14]

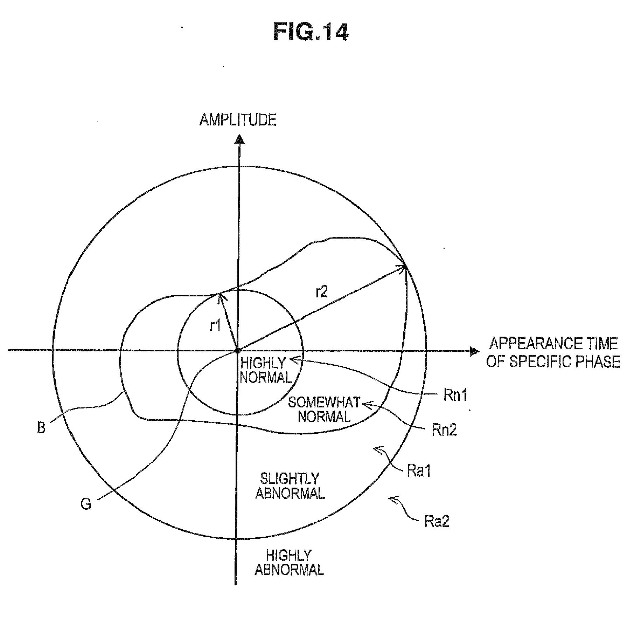

   Fig. 14 is a diagram illustrating an example of an estimation criterion of a feature quantity of an eardrum temperature based on statistical processing.
[Fig. 15]

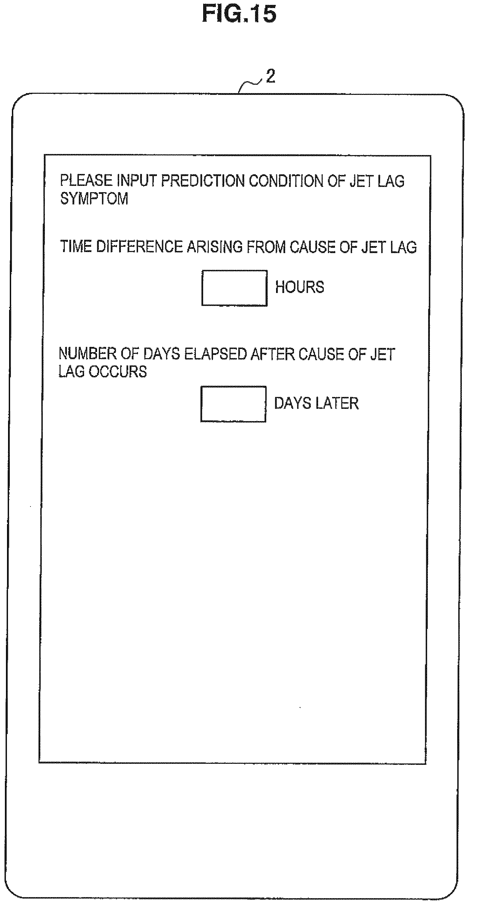

   Fig. 15 is a diagram illustrating an example of a questionnaire used to acquire a symptom prediction condition.
[Fig. 16]

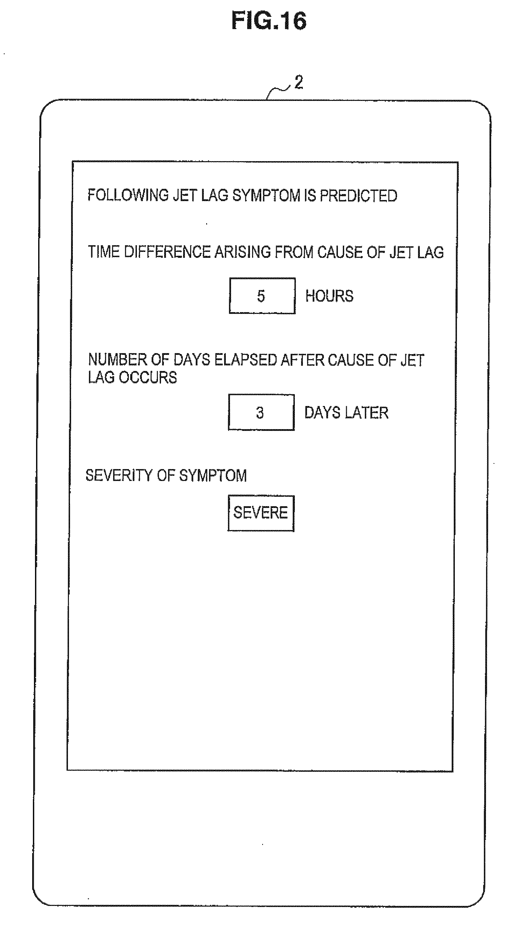

   Fig. 16 is a diagram illustrating an example of a symptom prediction result.
[Fig. 17]

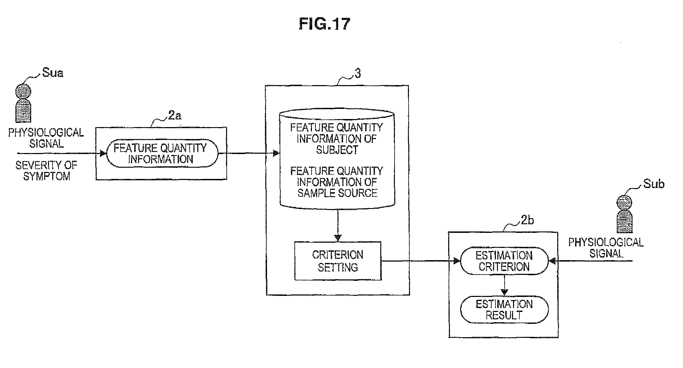

   Fig. 17 is a diagram illustrating an example of an estimation system in which an estimation criterion can be set in view of feature quantities of a plurality of subj ects.
[Fig. 18]

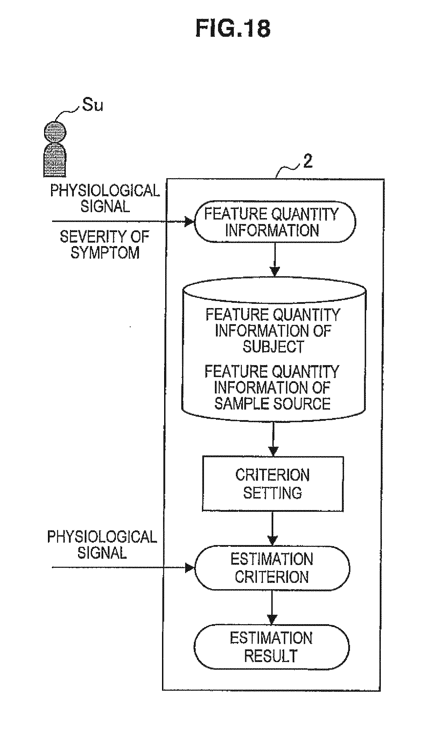

   Fig. 18 is a diagram illustrating an example of an estimation system in which an estimation criterion can be set in view of a feature quantity of a subject.
[Fig. 19]

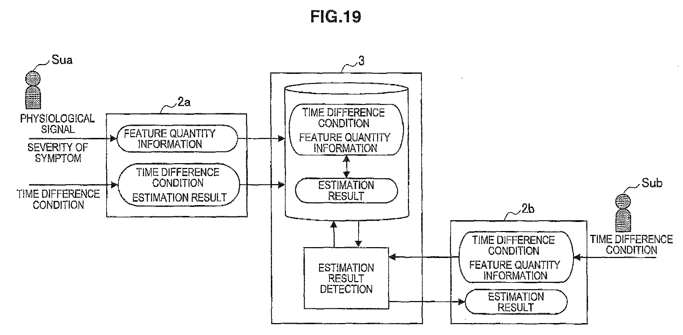

   Fig. 19 is a diagram illustrating an example of an estimation system in which a symptom estimation history can be shared between a plurality of estimation devices.

### Description of Embodiments

Hereinafter, preferred embodiments of the present disclosure will be described in detail with reference to the appended drawings. Note that, in this description and the drawings, elements that have substantially the same function and structure are denoted with the same reference signs, and repeated explanation is omitted.

### [1. Configuration of estimation system]

First of all, an estimation system for a jet lag symptom (hereinafter, also referred to as an "estimation system") according to an embodiment of the present disclosure will be described with reference to Figs. 1 to 5. Fig. 1 is a diagram illustrating an overall configuration of an estimation system according to an embodiment of the present disclosure.

As illustrated in Fig. 1, an estimation system includes a setting device 1 that sets an estimation criterion EC used for estimation of jet lag symptoms and an estimation device 2 that estimates jet lag symptoms using the estimation criterion EC and generates an estimation result ER. The estimation criterion EC is set based on a plurality of samples Sa that differ in jet lag symptoms, and used for estimation of jet lag symptoms of a subject Su.

Figs. 2 and 3 are flowcharts illustrating an overall process of the estimation system. In the estimation system, the setting device 1 performs a setting process of the estimation criterion EC as illustrated in Fig. 2, and the estimation device 2 performs an estimation process of jet lag symptoms as illustrated in Fig. 3.

First of all, the setting process of the estimation criterion EC will be described with reference to Fig. 2. The estimation criterion EC is set to each of the physiological index of the core body temperature system and the physiological index of the activity (sleep and wakefulness) system. For each of a plurality of samples Sa that differ in a severity of a jet lag symptom, the setting device 1 measures a physiological signal of the sample Sa (steps S11 and S21), derives a physiological index from the physiological signal (S12 and S22), extracts a feature quantity from the physiological index (S13 and S23), and acquires a severity of symptoms (S14 and S24).

The core body temperature system index is an index that is longer in an adapting time of a biological rhythm than the activity system index. For example, there are an eardrum temperature, a mouth temperature, and a content of melatonin or cortisol in blood. Meanwhile, the activity system index is an index that is shorter in the adapting time of the biological rhythm than the core body temperature system index. For example, there are activity, a pulse rate, an augmentation index (AI) value, and a blood pressure value.

Examples of the feature quantity of the physiological index include an eardrum temperature, a mouth temperature, a content of melatonin or cortisol, a pulse rate, an AI value, and a blood pressure value based on an appearance time of a specific phase (a maximum amplitude, a minimum amplitude, or the like), an amplitude quantity (maximum, minimum, average, or the like), etc. Further activity is based on a sleep onset time and a sleep offset time, etc. The plurality of samples Sa are a plurality of sample sources Sa different from the subject Su serving as a symptom estimation target, and the same sample source Sa may be used as two or more samples Sa. Further, the plurality of samples Sa may include the subject Su, but the description will proceed with an example in which the plurality of samples Sa are the plurality of sample sources Sa.

The setting device 1 classifies the feature quantity of the physiological index in the plurality of samples Sa according to a severity of symptoms (S15 and S25), specifies a feature quantity range R according to a classification of the severity of symptoms (S16 and S26), and sets the estimation criterion EC (S 17 and S27). The estimation criterion EC is set to each of the feature quantity of the core body temperature system index and the feature quantity of the activity system index.

Here, for example, the feature quantity of the physiological index is classified into two phases of a feature quantity of a "normal" sample Sa (a sample Sa having no symptoms) and a feature quantity of an "abnormal" sample Sa (a sample Sa having symptoms based on the severity of symptoms acquired from the sample Sa). The feature quantity of the physiological index may be classified into three or more phases, but the description will proceed with an example in which the feature quantity of the physiological index is classified into two phases.

Further, the feature quantity of the physiological index in the plurality of samples Sa is classified according to the severity of symptoms, and the feature quantity range Rn including many feature quantities of the "normal" samples Sa and the feature quantity range Ra including many feature quantities of the "abnormal" sample Sa are specified. Thus, the estimation criterion EC including the "normal" feature quantity range Rn and the "abnormal" feature quantity range Ra is set.

Next, the estimation process of the jet lag symptoms will be described with reference to Fig. 3. The estimation device 2 measures the physiological signal of the subject Su (steps S31 and S41), drives the physiological index from the physiological signal (S32 and S42), and extracts the feature quantity from the physiological index (S33 and S43). Here, as the feature quantity of the physiological index, the feature quantities of the core body temperature system index and the activity system index which are the same as the feature quantity extracted at the time of the setting process are extracted.

The estimation device 2 determines the feature quantity range R of the estimation criterion EC in which the feature quantity of the physiological index of the subject Su is included (S34 and S44), and estimates symptoms of the subject Su based on the determination result of the feature quantity (S51). Here, the feature quantity range R in which the feature quantity of the physiological index is included is determined for each of the feature quantity of the core body temperature system index and the feature quantity of the activity system index.

For example, it is determined that the feature quantity of the core body temperature system index is included in the "abnormal" feature quantity range Ra, and the feature quantity of the activity system index is included in the "normal" feature quantity range Rn. In this case, it is understood that the biological rhythm of the activity system adapts to actual living hours but does not adapt to the biological rhythm of the core body temperature system. As a result, severe jet lag symptoms in which the biological rhythm of the core body temperature system is not synchronized with the biological rhythm of the activity system are estimated.

Fig. 4 is a block diagram illustrating a functional configuration of the setting device 1. As illustrated in Fig. 4, the setting device 1 includes a signal measuring unit 11, an index deriving unit 12, a feature quantity extracting unit 13, a sample information acquiring unit 14, a criterion setting unit 15, and a storage unit 16. In the components excluding the sample information acquiring unit 14, the process is performed on each of the core body temperature system index and the activity system index.

The signal measuring unit 11 measures the physiological signal of the sample Sa using various kinds of sensors. For example, an eardrum temperature and a mouth temperature are measured using a temperature sensor, a pulse rate and a blood pressure value are measured using a pressure sensor, and activity is measured using an acceleration sensor. For example, a physiological signal is measured at predetermined measurement intervals during a predetermined duration throughout a measurement period of one night or the like. The measured physiological signal is converted into physiological data.

The index deriving unit 12 derives a physiological index from the physiological data. In the physiological data, a specific frequency component is removed using a filter or the like, or data at the time of non-resting is dismissed using an output of an acceleration sensor or the like. The physiological data is converted into other physiological indices (for example, converted from a pulse wave into an AI value) as necessary, and derived as an average value of physiological indices during a predetermined duration (for example, several seconds to several minutes).

The feature quantity extracting unit 13 extracts a feature quantity from the derived physiological index. For example, a feature quantity of an eardrum temperature, a mouth temperature, a pulse rate, an AI value, or a blood pressure value is extracted such that a function of a circadian cycle suitable for time-series data of the derived physiological index is obtained, and an appearance time of a specific phase (a maximum amplitude, a minimum amplitude, or the like) or an amplitude quantity (maximum, minimum, average, or the like) is obtained using the obtained function. Further, a feature quantity of activity is extracted such that the boundary between an active state and a sleep state is specified from the time-series data of the derived activity, for example, using an algorithm of Cole-Kripke (Reference 6) or the like.

The sample information acquiring unit 14 acquires sample information representing a severity of symptoms, a living style (a morning style, a night style, an intermediate style, or the like), and the like from the sample Sa. The sample information is acquired through an input device such as a key, a button, or a touch panel.

The criterion setting unit 15 sets the estimation criterion EC to be used for symptom estimation from the extracted feature quantity. The estimation criterion EC is set by classifying the feature quantity of the physiological index in a plurality of samples Sa according to a severity of symptoms and specifying the feature quantity range R according to a classification of the severity of symptoms. For example, the feature quantity range R is specified using a machine learning algorithm such as a support vector machine or boosting. For example, the estimation criterion EC may be set as the estimation criterion EC for a subject of each of a morning style, a night style, and an intermediate style based on the sample information representing a living style.

The storage unit 16 stores time-series data of the physiological signal or the physiological index of the sample Sa, the feature quantity, the sample information, the estimation criterion EC, and the like. The estimation criterion EC may be shared with the estimation device 2 or may be provided to the estimation device 2 through a communication line or a recording medium.

Fig. 5 is a block diagram illustrating a functional configuration of the estimation device 2. As illustrated in Fig. 5, the estimation device 2 includes a signal measuring unit 21, an index deriving unit 22, a feature quantity extracting unit 23, a subject information acquiring unit 24, a symptom estimating unit 25, a symptom notifying unit 26, a storing unit 27, a time difference condition acquiring unit 28, a history storing unit 29, and a history extracting unit 30. In the signal measuring unit 21, the index deriving unit 22, and the feature quantity extracting unit 23, processing is performed on each of the core body temperature system index and the activity system index.

The signal measuring unit 21, the index deriving unit 22, and the feature quantity extracting unit 23 function similarly to the corresponding components of the setting device 1. The feature quantity extracting unit 23 extracts the feature quantity from the physiological index derived from the subject Su. The subject information acquiring unit 24 acquires subject information representing a living style from the subject Su.

The symptom estimating unit 25 determines the feature quantity range R of the estimation criterion EC in which the feature quantity of the physiological index of the subject Su is included. Further, the symptom estimating unit 25 estimates the symptoms of the subject Su based on the determination result of the feature quantity of each of the core body temperature system index and the activity system index. Further, the determination of the feature quantity may be performed using the estimation criterion EC corresponding to the living style of the subject Su acquired as the subject information.

The symptom notifying unit 26 notifies the user of the symptom estimation result ER and a symptom estimation history which will be described later. The symptom notification is made as visual information and/or auditory information. The storing unit 27 stores the estimation criterion EC, time-series data of a measured value or a derived value of the physiological index of the subject Su, and the feature quantity. The storing unit 27 stores the estimation criterion EC shared with the setting device 1 and the estimation criterion EC acquired from the setting device 1 through a communication line or a recording medium.

The time difference condition acquiring unit 28 acquires a time difference condition representing a time difference arising from the cause (moving, shift working, or the like) of jet lag and the number of days elapsed after the cause of jet lag occurs. The time difference condition may be acquired from the subject Su or may be acquired through a GPS device, an acceleration sensor, or the like.

The history storing unit 29 stores time-series data of a derived value of the physiological index, the feature quantity, the estimation result of the feature quantity, the symptom estimation result ER, and the like in association with the time difference condition acquired from the subject Su as the symptom estimation history. Fig. 5 illustrates an example in which information is stored through the storing unit 27, but information may be stored without the storing unit 27. Based on a prediction condition (time difference condition) designated by the subject Su, the history extracting unit 30 extracts the estimation result ER satisfying the prediction condition among symptom estimation histories, and supplies the estimation result ER to the symptom notifying unit 26.

Each of the components of the setting device 1 and the estimation device 2 is configured as hardware such as a circuit logic and/or software such as a program. A component configured as software is implemented as a program on a CPU (not shown).

The estimation system may be configured such that the setting device 1 is integrated with the estimation device 2. For example, the estimation system or the estimation device 2 may be configured as a portable music player, a mobile telephone, a portable information terminal, or the like. In this case, in order to measure the physiological index, a sensor such as a temperature sensor or an acceleration sensor may be mounted in an earphone. Further, the symptom estimation result ER may provide the subject Su with a notification through a display device such as a liquid crystal display or an output device such as a speaker instead of the estimation device 2.

Alternatively, the estimation system may be configured such that the setting device 1 is separated from the estimation device 2. In this case, the estimation criterion EC and the symptom estimation result ER which are used for symptom estimation are transmitted and received between the setting device 1 and the estimation device 2 through a communication line or a recording medium. Further, the estimation device 2 may be configured to output the symptom estimation result ER to another user terminal or the like.

### [2. Operation of estimation system]

Next, an operation of the estimation system will be described with reference to Figs. 6 to 16. The following description will proceed with an example in which the feature quantity of the physiological index is classified into two phases of the feature quantity of the "normal" sample Sa and the feature quantity of the "abnormal" sample Sa using an eardrum temperature and activity as the core body temperature system index and the activity system index, respectively.

### (Setting process of estimation criterion EC)

Fig. 6 is a flowchart illustrating an operation of the setting device 1. First of all, for each of a plurality of samples Sa that differ in a severity of symptoms, the setting device 1 measures a physiological signal of the sample Sa, derives a physiological index from the physiological signal, extracts a feature quantity from the physiological index, and acquires subject information representing a severity of symptoms. In the following, an operation of extracting a feature quantity of a physiological index of one(1) sample Sa will be described.

The signal measuring unit 11 measures an eardrum temperature and activity of the sample Sa at predetermined measurement intervals during a predetermined duration throughout a predetermined measurement period (step S61). The measured values of the eardrum temperature and the activity are stored in the storage unit 16 as time-series data. For example, the measurement period is one night, for example, the measurement interval is several minutes to several tens of minutes, and for example, the duration is several tens of seconds to several minutes. The measurement is performed in a state in which the sample Sa is resting while avoiding activity for one or two hours after meals. The eardrum temperature and the activity may be measured at the same point in time or may be measured at different points in time.

The index deriving unit 12 calculates an average value of the eardrum temperature and an average value of the activity during the duration as the physiological index using the time-series data of the measured values (S62). The derived values of the eardrum temperature and the activity are stored in the storage unit 16 as time-series data. Further, when the physiological index is derived, an abnormal value such as nonconsecutive data or data at the time of non-resting is dismissed in the time-series data of the measured value. Further, data conversion of converting, for example, a pulse wave into an AI value (corresponding to an inflection point of a quadratic differential of a pulse wave) is executed on the time-series data of the measured value according to a physiological index.

The feature quantity extracting unit 13 extracts the feature quantity of the eardrum temperature and the feature quantity of the activity as the feature quantity of the physiological index using the time-series data of the derived value (S63). The feature quantities of the eardrum temperature and the activity are stored in the storage unit 16 in associated with the sample Sa.

Fig. 7 is a diagram illustrating an example of the feature quantity of the eardrum temperature and the feature quantity of the activity. As illustrated in Fig. 7, a function of a circadian cycle suitable for the time-series data of the derived value is obtained, and the feature quantity of the eardrum temperature is extracted, for example, as a combination of an appearance time (phase) of a minimum amplitude and an amplitude quantity thereof. The feature quantity may be extracted, for example, as a combination of two or more values of an appearance time of a specific phase (a maximum amplitude, a minimum amplitude, or the like) and an amplitude quantity (maximum, minimum, average, or the like). The feature quantity of the activity is extracted such that the boundary between the active state and the sleep state is specified as the sleep onset time from the time-series data of the derived value based on the distribution state.

The sample information acquiring unit 14 acquires the sample information representing a severity of symptoms from the sample source Sa using a questionnaire for the sample source Sa (S64). The sample information is stored in the storage unit 16 in association with the sample Sa. Alternatively, sample information representing a living style may be acquired from the sample source Sa. The living style of the sample Sa is designated as any one of a morning style, a night style, an intermediate style, and the like using a questionnaire for the sample Sa.

Fig. 8 is a diagram illustrating an example of the questionnaire for the sample source Sa. As illustrated in Fig. 8, for physiological indices other than the activity, for example, sample information is acquired using a questionnaire including items of insomnia/drowsiness, working efficiency, motivation/appetite, fatigue, and activity. The sample source Sa performs a five-point evaluation on each item, giving five points to a good state and one point to a bad state. Further, for the activity, an ordinary sleep onset time of the sample source Sa is acquired as sample information.

Next, the setting device 1 classifies the feature quantity of the physiological index in a plurality of samples Sa according to a severity of symptoms, specifies the feature quantity range R according to a classification of the severity of symptoms, and sets the estimation criterion EC.

The criterion setting unit 15 classifies the feature quantity of the physiological index in a plurality of samples Sa into the feature quantity of the "normal" sample Sa and the feature quantity of the "abnormal" sample Sa according to the severity of symptoms based on the sample information representing the severity of symptoms (S65). The classification result of the feature quantity is stored in the storage unit 16 in association with the sample Sa.

For the physiological index other than the activity, based on a total score of the questionnaire items, for example, the physiological index of a total score of 16 or more is classified into the feature quantity of the "normal" sample Sa, and the physiological index of a total score of 15 or less is classified into the feature quantity of the "abnormal" sample Sa. Further, the activity in which the difference between an ordinary sleep onset time (sample information) and an actual sleep onset time (the feature quantity of the activity) is within a predetermined time (for example, one hour) is classified into the feature quantity of the "normal" sample Sa, and the activity in which the difference exceeds the predetermined time is classified into the feature quantity of the "abnormal" sample Sa.

The criterion setting unit 15 specifies the "normal" feature quantity range Rn and the "abnormal" feature quantity range Ra based on the classification result of the feature quantity (S66), and sets the estimation criterion EC (S67). Further, the estimation criterion EC of each living style is set using a feature quantity space of each living style. The specifying result of the feature quantity range R is stored in the storage unit 16 as the estimation criterion EC.

Fig. 9 is a diagram illustrating an example of specifying the feature quantity range R on the feature quantity of the eardrum temperature and the feature quantity of the activity. Fig. 9 illustrates an example of a feature quantity space including the feature quantity distribution, the shape of the feature quantity range R, and the like. As illustrated in Fig. 9, for the eardrum temperature (the physiological index of the activity), for example, the estimation criterion EC is obtained using a feature quantity space (plane) having an appearance time of a specific phase as a first dimension and an amplitude quantity as a second dimension. For the activity, the estimation criterion EC is obtained using a feature quantity space (line) having a sleep onset time as a first dimension. Further, when the feature quantity of the physiological index includes a combination of n values, the estimation criterion EC is obtained using a feature quantity space of first to n-th dimensions.

Fig. 9 illustrates the feature quantity space (plane and line) in which the feature quantity of the "normal" sample Sa is plotted as an "O" mark, and the feature quantity of the "abnormal" sample Sa is plotted as an "X" mark. In the feature quantity space, a boundary B between a range Rn including many of the feature quantities of the "normal" samples Sa and a range Ra including many of feature quantities of the "abnormal" samples Sa is specified using a machine learning algorithm. As a result, the estimation criterion EC including the "normal" feature quantity range Rn and the "abnormal" feature quantity range Ra is set.

### (Symptom estimation process)

Fig. 10 is a flowchart illustrating an operation of the estimation device 2. First of all, the estimation device 2 measures the physiological signal of the subject Su, derives the physiological index from the physiological signal, and extracts the feature quantity from the physiological index.

The signal measuring unit 21 measures the eardrum temperature and the activity of the subject Su at predetermined measurement intervals during a predetermined duration throughout a predetermined measurement period (step S71). The measured values of the eardrum temperature and the activity are stored in the storing unit 27 as time-series data. The index deriving unit 22 calculates an average value of the eardrum temperature and an average value of the activity during the duration as the physiological index using the time-series data of the measured values (S72). The derived values of the eardrum temperature and the activity are stored in the storage unit 27 as time-series data.

The feature quantity extracting unit 23 extracts the feature quantity (for example, an appearance time of a minimum amplitude and an amplitude value thereof) of the eardrum temperature and the feature quantity (the sleep onset time) of the activity as the feature quantity of the physiological index using the time-series data of the derived value (S73). The feature quantities of the eardrum temperature and the activity are stored in the storing unit 27. The subject information acquiring unit 24 may acquire the sample information representing a living style from the sample Sa. The living style is designated as any one of a morning style, a night style, an intermediate style, and the like using a questionnaire for the subject Su.

Next, the estimation device 2 determines the feature quantity range R of the estimation criterion EC in which the feature quantity of the physiological index of the subject Su is included, and estimates the symptom of the subject Su based on the determination result of the feature quantity.

The symptom estimating unit 25 reads the estimation criterion EC of the feature quantity of the eardrum temperature and the estimation criterion EC of the feature quantity of the activity from the storing unit 27. The symptom estimating unit 25 determines which of the "normal" feature quantity range Rn and the "abnormal" feature quantity range Ra the feature quantity of the subject Su is included in on each of the eardrum temperature and the activity (S74).

Fig. 11 is a diagram illustrating an example of determining the feature quantity on the feature quantity of the eardrum temperature and the feature quantity of the activity. In the example illustrated in Fig. 11, a feature quantity of a subject A is determined to be included in the "normal" feature quantity range Rn of the estimation criterion EC of the eardrum temperature, and feature quantities of subjects B and C are determined to be included in the "abnormal" feature quantity range Ra. Similarly, the feature quantities of the subjects A and B are determined to be included in the "normal" feature quantity range Rn of the estimation criterion EC of the activity, and the feature quantity of subject C is determined to be included in the "abnormal" feature quantity range Ra.

The symptom estimating unit 25 estimates a severity of symptoms of the subject Su, for example, using Table 1, based on the determination result of the feature quantities of the eardrum temperature and the activity (S75).

**[Table 1]**

| Core body temperature system index | Activity system index | Severity of symptoms |
|---|---|---|
| Abnormal | Normal | Severe |
| Abnormal | Abnormal | Moderate |
| Normal | Normal | Mild (or no symptoms) |

In other words, when the eardrum temperature is used as the core body temperature system index and the activity is used as the activity system index, if the feature quantity of the eardrum temperature is determined to be "abnormal" and the feature quantity of the activity is determined to be "normal," symptoms are estimated to be severe. Similarly, when the feature quantity of the eardrum temperature is determined to be "abnormal" and the feature quantity of the activity is determined to be "abnormal," symptoms are estimated to be moderate, and when the feature quantity of the eardrum temperature is determined to be "normal" and the feature quantity of the activity is determined to be "normal," symptoms are estimated to be mild, or no symptoms are estimated. Thus, it is possible to objectively determine the presence or absence of symptoms and quantitatively estimate a severity of symptoms.

The symptom notifying unit 26 notifies the subject Su of the symptom estimation result ER (S76). The symptom estimation result ER includes at least information representing a severity of symptoms such as "severe," "moderate," and "mild" and may further include the determination result of the feature quantities of the eardrum temperature and the activity. Further, the symptom estimation result ER may include the time-series data of the measured values or the derived values of the eardrum temperature and the activity used for estimation of symptoms, the feature quantity, and the like. In addition, the symptom estimation result ER may be displayed in comparison with an estimation history of a specific date and time, an average estimation history, or the like.

Fig. 12 is a diagram illustrating an example of notifying of the symptom estimation result ER. In the example illustrated in Fig. 12, the feature quantity of the eardrum temperature is determined to be "abnormal," and the feature quantity of the activity is determined to be "normal," and thus a severity of symptoms is estimated to be "severe."

In this case, it is understood that as the feature quantity of the core body temperature system index is included in the "abnormal" feature quantity range Ra and the feature quantity of the activity system index is included in the "normal" feature quantity range Rn, the biological rhythm of the activity system adapts to the actual living hours and the like, but the biological rhythm of the core body temperature system does not adapt thereto. Thus, the biological rhythm of the core body temperature system is not synchronized with the biological rhythm of the activity system, and severe jet lag symptoms are estimated.

### (Another setting example 1 of estimation criterion EC)

In the above description, the estimation criterion EC is set to classify the feature quantities of the physiological indices into two phases of severity of the feature quantity of the "normal" sample Sa and the feature quantity of the "abnormal" sample Sa. However, the estimation criterion EC may be set to classify the feature quantity of the physiological index into three or more phases. Thus, the symptoms of the subject Su are estimated in further detail than in the two-phase classification. The following description will proceed with an example in which the feature quantity of the physiological index is classified into four phases based on the sample information or statistical processing.

First of all, an example in which the feature quantities of the physiological indices are classified into four phases based on the sample information will be described. The sample information acquiring unit 14 acquires sample information representing a severity of symptoms from each sample Sa using the above-described questionnaire. The criterion setting unit 15 classifies the feature quantity of the physiological index in a plurality of samples Sa based on the sample information.

For the physiological index of the activity, based on a total score of the questionnaire items, for example, the physiological index of a total score of 21 to 25 is classified into the feature quantity of the sample Sa of "highly normal," the physiological index of a total score of 16 to 20 is classified into the feature quantity of the sample Sa of "somewhat normal," the physiological index of a total score of 10 to 15 is classified into the feature quantity of the sample Sa of "slightly abnormal," and the physiological index of a total score of 5 to 9 is classified into the feature quantity of the sample Sa of "highly abnormal." The activity in which the difference between the ordinary sleep onset time and the actual sleep onset time is less than one hour is classified into the feature quantity of the sample Sa of "highly normal," the activity in which the difference is one hour or more and less than two hours is classified into the feature quantity of the sample Sa of "somewhat normal," the activity in which the difference is two hours or more and less than four hours is classified into the feature quantity of the sample Sa of "slightly abnormal," and the activity in which the difference is four hours or more is classified into the feature quantity of the sample Sa of "highly abnormal."

Then, the criterion setting unit 15 sets the estimation criterion EC using the feature quantity space for each physiological index based on the classification result of the feature quantity. In other words, feature quantity ranges Rn1, Rn2, Ra1, and Ra2 of "highly normal," "somewhat normal," "slightly abnormal," and "highly abnormal" are specified using the machine learning algorithm. Fig. 13 is a diagram illustrating an example of the estimation criterion EC of the feature quantity (time, amplitude) of the eardrum temperature based on the sample information. Further, Fig. 13 illustrates an example of a feature quantity space including the shape of the feature quantity range R and the like.

### (Another setting example 2 of estimation criterion EC)

Next, an example in which the feature quantities of the physiological indices are classified into four phases based on statistical processing when it is difficult to classify a feature quantity based on the sample information will be described. The sample information acquiring unit 14 acquires the sample information representing the severity of symptoms from each sample Sa using the above-described questionnaire, similarly to the example based on the sample information. The criterion setting unit 15 classifies the feature quantities of the physiological indices of a plurality of samples Sa into two phases, that is, "normal" and "abnormal," based on the sample information.

The criterion setting unit 15 sets the estimation criterion EC using the feature quantity space for each physiological index based on the classification result of the feature quantity. First of all, the boundary B between the range Rn (the "normal" range Rn) of the feature quantity of the "normal" sample Sa and the range Ra (the "abnormal" range Rn) of the feature quantity of the "abnormal" sample Sa is specified using the machine learning algorithm. Next, a gravity center G of the "normal" range Rn is obtained.

Further, when it is in a space (plane) having a minimum distance r1 from the gravity center of the "normal" range Rn to the boundary between the "normal" range Rn and the "abnormal" range Ra, it is classified into "highly normal," and when it is within a range outside "highly normal" within the "normal" range Rn, it is classified into "somewhat normal." Further, when it is in a space (plane) having a maximum distance r2 from the gravity center of the "normal" range Rn to the boundary between the "normal" range Rn and the "abnormal" range Rn, it is classified into "slightly abnormal," and when it is within a range outside that range, it is classified into "highly abnormal." Thus, feature quantity ranges Rn1, Rn2, Ra1, and Ra2 of "highly normal," "somewhat normal," "slightly abnormal," and "highly abnormal" are specified. Fig. 14 is a diagram illustrating an example of the estimation criterion EC of the feature quantity (time, amplitude) of the eardrum temperature based on the statistical processing. Further, Fig. 14 illustrates an example of a feature quantity space including the shape of the feature quantity range R and the like.

When the four-phase estimation criterion EC is set to the core body temperature system index and the activity system index, a severity of symptoms of the subject Su is estimated, for example, based on Table 2, from the determination result of the feature quantity of each physiological index. Further, it is understood that as a value of a severity of symptoms increases, symptoms are severer. Thus, as a severity of symptoms is classified in further detail, quantitative evaluation can be made in further detail.

**[Table 2]**

| Core body temperature system index | Activity system index | Severity of symptoms |
|---|---|---|
| Highly normal | Highly abnormal | 1 (severe) |
| Somewhat normal | Highly abnormal | 2 (severe) |
| Highly normal | Slightly abnormal | 3 (severe) |
| Somewhat normal | Slightly abnormal | 4 (severe) |
| Slightly abnormal | Highly abnormal | 5 (moderate) |
| Highly abnormal | Highly abnormal | 6 (moderate) |
| Slightly abnormal | Slightly abnormal | 7 (moderate) |
| Highly normal | Somewhat normal | 8 (mild) |
| Somewhat normal | Somewhat normal | 9 (mild) |
| Highly normal | Highly normal | 10 (mild, no symptom) |

When the four-phase estimation criterion EC is set to either of the core body temperature system index and the activity system index, a severity of symptoms of the subject Su is estimated, for example, based on the following tables from the determination result of the feature quantity of each physiological index. Table 3 is an example in which the four-phase estimation criterion EC is set to the core body temperature system index, and the two-phase estimation criterion EC is set to the activity system index. Table 4 is an example in which the two-phase estimation criterion EC is set to the core body temperature system index, and the four-phase estimation criterion EC is set to the activity system index.

**[Table 3]**

| Core body temperature system index | Activity system index | Severity of symptoms |
|---|---|---|
| Highly abnormal | Normal | 1 (severe) |
| Slightly abnormal | Normal | 2 (severe) |
| Highly abnormal | Abnormal | 3 (moderate) |
| Slightly abnormal | Abnormal | 4 (moderate) |
| Somewhat normal | Abnormal | 5 (mild) |
| Highly normal | Normal | 6 (mild, no symptom) |

**[Table 4]**

| Core body temperature system index | Activity system index | Severity of the symptoms |
|---|---|---|
| Abnormal | Highly normal | 1 (severe) |
| Abnormal | Somewhat normal | 2 (severe) |
| Abnormal | Highly abnormal | 3 (moderate) |
| Abnormal | Slightly abnormal | 4 (moderate) |
| Normal | Slightly normal | 5 (mild) |
| Normal | Highly normal | 6 (mild, no symptom) |

### (Symptom prediction process)

Next, a method of predicting symptoms using the symptom estimation history will be described with reference to Figs. 15 and 16. In the symptom estimation process, the time difference condition acquiring unit 28 acquires the time difference condition representing a time difference arising from the cause of jet lag and the number of days elapsed after the cause of jet lag occurs. The time difference arising from the cause of jet lag is a moving time difference, a shift time, or the like, and the number of days elapsed after the cause of jet lag occurs is the number of days elapsed after movement involving a time difference, shift working, or the like occurs.

The estimation device 2 measures the physiological signal of the subject Su, derives the physiological index, extracts the feature quantity, and determines the feature quantity of the physiological index to estimate symptoms as described above. Here, the time-series data of the derived value of the physiological index, the feature quantity, the determination result of the feature quantity, the symptom estimation result ER, and the like are stored in the history storing unit 29 in association with the subject Su and the time difference condition as the symptom estimation history. Thus, the symptom estimation history is accumulated in the history storing unit 29 in a state in which the symptom estimation history can be referred to based on the subject Su and the time difference condition.

In order to predict a symptom, the time difference condition acquiring unit 28 acquires the time difference condition designating a time difference arising from the cause of jet lag and the number of days elapsed after the cause of jet lag occurs from the subject Su as a symptom prediction condition. Fig. 15 is a diagram illustrating an example of a questionnaire used to acquire the symptom prediction condition (the time difference condition).

When the time difference condition is acquired, the history extracting unit 30 extracts estimation history corresponding to the time difference condition from the estimation history stored in the history storing unit 29. The symptom notifying unit 26 displays the extracted estimation history. Fig. 16 is a diagram illustrating an example of the symptom prediction result. In the example of Fig. 16, for example, severe symptoms are predicted on a third day after movement involving a time difference of five hours is made. Thus, for example, the subject Su can predict symptoms according to the number of days elapsed after movement before and after movement involving a time difference.

### [3. Modified example of estimation system]

Next, a modified example of the estimation system will be described with reference to Figs. 17 to 19. The modified example of the estimation system relates to a method of setting the estimation criterion EC and a method of using the estimation history. The following description will proceed under the assumption that the estimation criterion EC is set such that the feature quantities of the physiological indices are classified into two phases of severity of the feature quantity of the "normal" sample Sa and the feature quantity of the "abnormal" sample Sa. However, the estimation criterion EC may be set according to another setting example 1 or 2 of the estimation criterion.

### (Another setting example 1 of estimation criterion EC)

The above description has been made in connection with the example in which symptoms of the subject Su are estimated using the estimation criterion EC previously stored in the estimation device 2. However, for example, the estimation criterion EC may be managed by a managing device 3 that is accessible from a plurality of estimation devices 2. Fig. 17 is a diagram illustrating an example of an estimation system in which the estimation criterion EC can be set in view of feature quantities of a plurality of subjects Su.

In this case, in the symptom estimation process, an estimation device 2a acquires information representing a severity of symptoms of a subject Sua, and classifies the feature quantity of the physiological index of the subject Sua into the feature quantity of the "normal" or "abnormal" sample Sa, similarly to the setting process of the estimation criterion EC. Then, the estimation device 2a transmits the classification result of the feature quantity and the feature quantity to the managing device 3 as feature quantity information. The managing device 3 manages the feature quantity information of the subject Sua on a database together with the feature quantity information of other subjects Su or other sample sources Sa

Meanwhile, when a symptom of a subject Sub is estimated, an estimation device 2b requests the managing device 3 to transmit the estimation criterion EC. According to a transmission request of the estimation criterion EC, the managing device 3 newly sets the estimation criterion EC based on the feature quantity information managed therein, and transmits the estimation criterion EC to the estimation device 2b. Similarly to the estimation process, the estimation criterion EC is set such that the feature quantity is plotted on the feature quantity space according to the classification result of the feature quantity, and the feature quantity range R is specified. The estimation device 2b extracts the feature quantity of the physiological index of the subject Sub, and estimates a symptom of the subject Sub using the new estimation criterion EC.

Thus, as the estimation criterion EC is set using the feature quantity information of a plurality of subjects Su, it is possible to implement the estimation system in which symptoms of the subject Su can be estimated without using the estimation criterion EC previously stored in the estimation device 2.

### (Another setting example 2 of estimation criterion EC)

The above description has been made in connection with the example in which the estimation criterion EC is set using the feature quantity information of a plurality of sample sources Sa different from the subject Su. However, the estimation criterion EC may be set in view of the feature quantity information of the subject Su. The feature quantity information of the subject Su may be managed by the estimation device 2 (for example, the estimation device 2 integrated with the setting device 1) capable of performing the setting process of the estimation criterion EC or may be managed by the managing device 3 that is accessible from the estimation device 2. In the following, the former case will be described. Fig. 18 is a diagram illustrating an example of an estimation system in which the estimation criterion EC can be set in view of the feature quantity of the subject Su.

In this case, in the symptom estimation process, the estimation device 2 acquires information representing a severity of symptoms of the subject Su, and classifies the feature quantity of the physiological index of the subject Su, similarly to the setting process of the estimation criterion EC. Then, the estimation device 2 newly sets the estimation criterion EC in view of the feature quantity information acquired in the previous estimation process together with the feature quantity information of the other sample sources Sa. Alternatively, the estimation criterion EC may be set using the feature quantity information of the subject Su himself or herself. In another estimation process, the estimation device 2 extracts the feature quantity of the physiological index of the same subject Su as in the previous estimation process, and estimates a symptom of the subject Su using the latest estimation criterion EC.

Thus, as the estimation criterion EC is set using the feature quantity information of the subject Su himself or herself, it is possible to estimate a symptom of the subject Su with a high degree of accuracy and to implement the estimation system dedicated for the subject Su.

### (Another use example of estimation history)

The above description has been made in connection with the example in which, in the symptom prediction process, symptoms of the subject Su are predicted using the symptom estimation history of the subject Su himself or herself. However, symptoms of the subject Su may be predicted using symptom estimation history of another subject Su. For example, the symptom estimation history of the subject Su is managed by the managing device 3 that is accessible from a plurality of estimation devices 2. Fig. 19 is a diagram illustrating an example of an estimation system in which the symptom estimation history can be shared between a plurality of estimation devices 2a and 2b.

In this case, in the symptom estimation process of the subject Sua, the estimation device 2 acquires information representing a severity of symptoms of the subject Sua, and classifies the feature quantity of the physiological index of the subject Sua, similarly to the setting process of the estimation criterion EC. Then, the estimation device 2a transmits the classification result of the feature quantity and the feature quantity to the managing device 3 as the feature quantity information. The estimation device 2a estimates symptoms of the subject Sua, and transmits the estimation result ER to the managing device 3 together with the time difference condition acquired from the subj ect Sua.

The managing device 3 manages information of the subject Sua together with information of other subjects Su. The managing device 3 manages the estimation result ER of a plurality of subjects Su on a database in association with the feature quantity information and the time difference condition.

In the symptom prediction process of the subject Sub, the estimation device 2b transmits the time difference condition and the feature quantity information of the subject Sub to the managing device 3 together with an estimation history transmission request. Further, the feature quantity information (the classification result of the feature quantity and the feature quantity) of the subject Sub is assumed to be obtained in advance in the symptom estimation process for the subject Sub.

According to the estimation history transmission request, the managing device 3 extracts an estimation history stored in association with a time difference condition and feature quantity information similar to the time difference condition and the feature quantity information of the subject Sub from the database. Then, the managing device 3 transmits the extracted estimation history to the estimation device 2b, and the estimation device 2b notifies the user of the estimation history acquired by an estimation history acquiring unit (not shown).

Accordingly, the symptom estimation history is shared with another subject Su that is similar in the feature quantity information, and thus the estimation system in which symptoms of the subject Su can be appropriately predicted can be implemented.

### (Reference List)

Reference 1: O'Connor, P.J.; Morgan, W.P. Athletic Performance Following Rapid Traversal of Multiple Time Zones-A Review. Sports Med. 1990, 10, 20-30.
Reference Literature 2: Klein, K.E.; Wegmann, H.-M. The Resynchronization of Human Circadian Rhythms After Transmeridian Flights as a Result of Flight Direction and Mode of Activity. In Chronobiology; Scheving, L.E., Halberg, F., Pauly, J.E., Eds.; Thieme Publ.: Stuttgart, 1974; 564-570.
Reference Literature 3: Winget, C.M.; De Roshia, C.M.; Holley, D.C. Circadian Rhythms and Athletic Performance. Med. Sci. Sports Exerc. 1985, 17, 498-516.
Reference 4: Reilly, T. (1998). Travel: Physiology, jet-lag, strategies. In: Encyclopedia of Sports Medicine and Science, T.D.Fahey (Editor). Internet Society for Sport Science: http://sportsci.org. 12 July 1998.
Reference 5: Tadao Hori; Shuichiro Shirakawa "Basic course, sleep improvement study"
Reference Literature 6: Cole RJ, Kripke DF, Gruen W, Mullaney DJ, Gillin JC. Automatic sleep/wake identification from wrist activity. Sleep. 1992; 15:461-9.

The preferred embodiments of the present disclosure have been described in detail above with reference to the accompanying drawings, whilst the present disclosure is not limited to the examples according to the present disclosure. A person skilled in the art may find various alternations and modifications within the scope of the appended claims, and it should be understood that they will naturally come under the technical scope of the present disclosure.

### Reference Signs List

- 1: setting device
- 2, 2a, 2b: estimation device
- 3: managing device
- 11, 21: signal measuring unit
- 12, 22: index deriving unit
- 13, 23: feature quantity extracting unit
- 14: sample information acquiring unit
- 15: criterion setting unit
- 16, 27: storage unit
- 24: subject information acquiring unit
- 25: symptom estimating unit
- 26: symptom notifying unit
- 28: time difference condition acquiring unit
- 29: history storing unit
- 30: history extracting unit
- Sa: sample
- Su, Sua, Sub: subject
- R, Rn, Ra, Rn1, Rn2, Ra1, Ra2: feature quantity range

## Claims

1. An estimation device for a jet lag symptom, comprising:
an acquiring unit that acquires an estimation criterion set in a manner that, for a plurality of samples that differ in a severity of a jet lag symptom, each of a feature quantity of a physiological index of a core body temperature system and a feature quantity of a physiological index of an activity system is classified according to the severity of the jet lag symptom, and a feature quantity range is specified according to a classification of the severity of the jet lag symptom;
an extracting unit that extracts the feature quantity of the physiological index of the core body temperature system and the feature quantity of the physiological index of the activity system on a subject; and
an estimating unit that determines which feature quantity range of the estimation criterion includes a feature quantity of a physiological index of the subject on each of the physiological index of the core body temperature system and the physiological index of the activity system, and estimates a jet lag symptom of the subject.

2. The estimation device for the jet lag symptom according to claim 1,
wherein the estimating unit estimates the severity of the jet lag symptom of the subject to be severe when the feature quantity of the physiological index of the core body temperature system is included in a feature quantity range of a sample having a jet lag symptom, and the feature quantity of the physiological index of the activity system is included in a feature quantity range of a feature quantity of a sample having no jet lag symptoms.

3. The estimation device for the jet lag symptom according to claim 1,
wherein the acquiring unit acquires an estimation criterion set in a manner that feature quantities of the plurality of samples are classified into a feature quantity range of a sample having a jet lag symptom and a feature quantity range of the sample having no jet lag symptoms, the feature quantities of the plurality of samples are classified into three or more according to the severity of the jet lag symptom based on a center of the feature quantity range of the sample having the jet lag symptom, and a sub feature quantity range is specified according to the severity of the jet lag symptom, and
the estimating unit determines which sub feature quantity range of the estimation criterion includes the feature quantity of the physiological index of the subject, and estimates the jet lag symptom of the subject.

4. The estimation device for the jet lag symptom according to claim 1,
wherein the acquiring unit acquires an estimation criterion set in a manner that the feature quantities of the plurality of samples are classified into three or more based on subjective evaluation on the severity of the jet lag symptom based on the sample, and a sub feature quantity range is specified according to the severity of the jet lag symptom, and
the estimating unit determines which sub feature quantity range of the estimation criterion includes the feature quantity of the physiological index of the subject, and estimates the jet lag symptom of the subject.

5. The estimation device for the jet lag symptom according to claim 3,
wherein the acquiring unit acquires an estimation criterion set in a manner that feature quantities of a plurality of samples including the subject are classified according to the severity of the jet lag symptom, and a feature quantity range according to a classification of the severity of the jet lag symptom is specified.

6. The estimation device for the jet lag symptom according to claim 3, further comprising:
an estimation history storing unit that stores an estimation result of the jet lag symptom in association with a time difference condition representing a condition of a time difference serving as a cause of the jet lag symptom; and
an estimation history extracting unit that extracts an estimation result suitable for a designated time difference condition from the stored estimation result of the jet lag symptom.

7. The estimation device for the jet lag symptom according to claim 3,
wherein the estimation device is connected to a managing device that acquires the feature quantity of the physiological index of the subject and a classification result of a feature quantity according to the jet lag symptom from a plurality of estimation devices, specifies a feature quantity range according to a classification of the severity of the jet lag symptom, and sets an estimation criterion, and
the acquiring unit acquires the estimation criterion set by the managing device.

8. The estimation device for the jet lag symptom according to claim 3,
wherein the estimation device is connected to a managing device that acquires a classification result of the feature quantity of the physiological index of the subject, a time difference condition representing a condition of a time difference serving as a cause of the jet lag symptom, and an estimation result of the jet lag symptom from a plurality of estimation devices, and manages the estimation result of the jet lag symptom in association with the classification result of the feature quantity and the time difference condition, and
the estimation device further comprises an estimation history acquiring unit that acquires an estimation result suitable for a designated time difference condition and a classification result of the feature quantity among the managed estimation results of the jet lag symptom.

9. The estimation device for the jet lag symptom according to claim 3,
wherein the extracting unit extracts the feature quantity of the physiological index of the core body temperature system and the feature quantity of the physiological index of the activity system on the plurality of samples that differ in the severity of the jet lag symptom,
the estimation device further comprises a setting unit that classifies the extracted feature quantity of the physiological index according to the severity of the jet lag symptom on each of the physiological index of the core body temperature system and the physiological index of the activity system, specifies a feature quantity range according to a classification of the severity of the jet lag symptom, and sets an estimation criterion, and
the acquiring unit acquires the estimation criterion set by the setting unit.

10. The estimation device for the jet lag symptom according to claim 3,
wherein the estimation criterion is set for each living style of the sample, and
the estimating unit estimates the jet lag symptom of the subject using an estimation criterion according to a living style of the subject.

11. The estimation device for the jet lag symptom according to claim 3, further comprising:
a notifying unit that notifies the subject of the estimation result of the jet lag symptom.

12. The estimation device for the jet lag symptom according to claim 3,
wherein the physiological index of the core body temperature system is an eardrum temperature, and the physiological index of the activity system is activity.

13. The estimation device for the jet lag symptom according to claim 1, further comprising:
a storage unit that stores the estimation criterion,
wherein the acquiring unit acquires the estimation criterion from the storage unit.

14. An estimation system for a jet lag symptom, comprising:
a setting device including
an extracting unit that extracts a feature quantity of a physiological index of a core body temperature system and a feature quantity of a physiological index of an activity system on a plurality of samples that differ in a severity of a jet lag symptom, and
a setting unit that classifies the extracted feature quantity of the physiological index according to the severity of the jet lag symptom on each of the physiological index of the core body temperature system and the physiological index of the activity system, specifies a feature quantity range according to a classification of the severity of the jet lag symptom, and sets an estimation criterion; and
an estimation device including
an extracting unit that extracts the feature quantity of the physiological index of the core body temperature system and the feature quantity of the physiological index of the activity system on a subject,
and
an estimating unit that determines which feature quantity range of the estimation criterion includes a feature quantity of a physiological index of the subject on each of the physiological index of the core body temperature system and the physiological index of the activity system, and estimates a jet lag symptom of the subject.

15. An estimation method for a jet lag symptom, comprising:
acquiring an estimation criterion set in a manner that, for a plurality of samples that differ in a severity of a jet lag symptom, each of a feature quantity of a physiological index of a core body temperature system and a feature quantity of a physiological index of an activity system is classified according to the severity of the jet lag symptom, and a feature quantity range is specified according to a classification of the severity of the jet lag symptom;
extracting the feature quantity of the physiological index of the core body temperature system and the feature quantity of the physiological index of the activity system on a subject; and
determining which feature quantity range of the estimation criterion includes a feature quantity of a physiological index of the subject on each of the physiological index of the core body temperature system and the physiological index of the activity system, and estimating a jet lag symptom of the subject.

16. A program for causing a computer to execute an estimation method for a jet lag symptom, the method including
acquiring an estimation criterion set in a manner that, for a plurality of samples that differ in a severity of a jet lag symptom, each of a feature quantity of a physiological index of a core body temperature system and a feature quantity of a physiological index of an activity system is classified according to the severity of the jet lag symptom, and a feature quantity range is specified according to a classification of the severity of the jet lag symptom,
extracting the feature quantity of the physiological index of the core body temperature system and the feature quantity of the physiological index of the activity system on a subject, and
determining which feature quantity range of the estimation criterion includes a feature quantity of a physiological index of the subject on each of the physiological index of the core body temperature system and the physiological index of the activity system, and estimating a jet lag symptom of the subject.

17. An estimation device for a jet lag symptom, comprising:
an acquiring unit that acquires an estimation criterion in which each of a feature quantity of a physiological index of a core body temperature system and a feature quantity of a physiological index of an activity system is classified according to a severity of a jet lag symptom, and a feature quantity range is specified according to a classification of the severity of the jet lag symptom;
an extracting unit that extracts the feature quantity of the physiological index of the core body temperature system and the feature quantity of the physiological index of the activity system from a user; and
an estimating unit that determines which feature quantity range of the estimation criterion includes a feature quantity of a physiological index of the user on each of the physiological index of the core body temperature system and the physiological index of the activity system, and estimates a jet lag symptom of the user.
